# EUROPÄISCHE PATENTSCHRIFT

(11) **EP 2 167 506 B1**
(45) Veröffentlichungstag und Bekanntmachung des Hinweises auf die Patenterteilung: **11.05.2016**
(21) Anmeldenummer: 08758517.0
(22) Anmeldetag: 14.05.2008
(51) Int. Cl.: C07D 489/08, A61K 31/485, A61P 25/04, A61P 1/10

(54) **VERFAHREN ZUR HERSTELLUNG VON N-METHYLNALTREXONBROMID**
METHOD FOR PRODUCING N-METHYLNALTREXONE BROMIDE
PROCÉDÉ DE PRODUCTION DE BROMURE DE N-MÉTHYLNALTREXON

(30) Priorität: 16.05.2007 WO PCT/EP2007/004365
(43) Veröffentlichungstag der Anmeldung: 31.03.2010
(73) Patentinhaber: Cilag AG, 8205 Schaffhausen (CH)
(72) Erfinder: WEIGL, Ulrich, 78247 Hilzingen (DE); SCHÄR, Pascal, CH-3007 Bern (CH); STUTZ, Alfred, CH-8037 Zürich (CH)
(74) Vertreter: Braun, André jr.
(86) Internationale Anmeldenummer: PCT/EP2008/003870
(87) Internationale Veröffentlichungsnummer: WO 2008/138605

(56) Entgegenhaltungen:
- WO-A-2004/043964
- WO-A-2008/034973
- WO-A2-2006/127899

## Beschreibung

Die vorliegende Erfindung betrifft ein Verfahren zur Herstellung von N-Methylnaltrexonbromid.

N-Methylnaltrexonbromid (CAS-Nr. 73232-52-7) ist eine bekannte pharmazeutisch wirksame Verbindung, die insbesondere zur Behandlung von postoperativem Ileus oder zur Behandlung von Opioid-induzierter Konstipation verwendet wird. N-Methylnaltrexonbromid wird auch als Naltrexon Methobromid bezeichnet und entspricht der chemischen Formel:

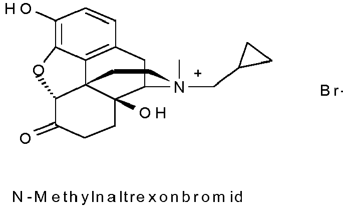

Die Verbindung N-Methylnaltrexonbromid ist in US 4,176,186 beschrieben. Die Verbindung wird beispielsweise hergestellt, indem man die freie Naltrexonbase mit Methylbromid umsetzt. Dieses Verfahren hat den Nachteil, dass die Reaktion in einem Druckgefäss in einem organischen Lösungsmittel, wie wasserfreies Aceton und/oder Dimethylformamid, über eine vergleichsweise lange Zeitdauer durchgeführt werden muss. Dabei entstehen zudem unerwünschte Nebenprodukte. In US 4,176,186 wird auch vorgeschlagen, die freie Naltrexonbase in einem ersten Schritt mit Methyliodid oder Dimethylsulfat zu methylieren und anschliessend das in der erhaltenen Verbindung vorhandene Anion mittels Chromatographie gegen das Bromidanion auszutauschen. Hierbei wird ein Bromid-beladener chromatographischer Ionenaustauscher verwendet. Der chromatographische Ionenaustausch ist jedoch für eine Herstellung von Methylnaltrexonbromid in grösseren Mengen ungeeignet, denn dieser ist sehr zeit-, volumen- und lösungsmittelintensiv und dadurch für den industriellen Gebrauch zu teuer. Es besteht deshalb ein Bedarf für ein Verfahren zur Herstellung von Methylnaltrexonbromid, welches in industriellem Massstab einfach und kostengünstig durchgeführt werden kann.

Es wurde nun gefunden, dass es überraschenderweise möglich ist, in an sich bekannter Weise die freie Naltrexonbase am Stickstoffatom mit einem geeigneten bromidfreien Methylierungsmittel zu methylieren und anschliessend das in der methylierten quaternären Verbindung vorhandene Anion in einfacher Weise und ohne Gebrauch chromatographischer Ionenaustauschtechnik gegen das gewünschte Bromidanion auszutauschen. Dabei erfolgt der Austausch der Anionen in einer Lösung oder Suspension der erhaltenen quaternären Verbindung in einem geeigneten polaren Lösungsmittel, wie beispielsweise Wasser, Alkohol oder einem Gemisch dieser Verbindungen, durch einfache Zugabe einer Bromidanionen enthaltenden Verbindung, vorzugsweise ebenfalls gelöst in einem polaren Lösungsmittel. Das derart gebildete Methylnaltrexonbromid fällt als Rohprodukt, gegebenenfalls nach zuvor erfolgter Abspaltung der Schutzgruppe an der phenolischen Gruppe, in einer Reinheit von mindestens 99% (Reinheit >99%) aus. Nach anschliessender Umkristallisation, beispielsweise aus Wasser/Methanol, stimmt der Wert des Bromidgehaltes zu mindestens 99.99% (>99.99%) mit der theoretisch berechneten Menge an Bromidanionen im Methylnaltrexonbromid überein. Dieses Verfahren ist geeignet zur kostengünstigen Herstellung grosser Mengen Methylnaltrexonbromid, da dieses ausschliesslich einfache Kristallisationsverfahren und keine aufwendige Ionenchromatographie beinhaltet.

Das erfindungsgemässe Verfahren beruht insbesondere auf der unterschiedlichen Löslichkeit des nach der Alkylierung vorliegenden Methylnaltrexonsalzes, im Vergleich zu Methylnaltrexonbromid. Da das Methylnaltrexonbromid in den verwendeten Lösungsmitteln deutlich weniger löslich ist, erfolgt der Austausch der Anionen praktisch quantitativ.

Die vorliegende Erfindung ist in den Ansprüchen definiert. Insbesondere betrifft die vorliegende Erfindung ein Verfahren zur Herstellung von N-Methylnaltrexonbromid, welches dadurch gekennzeichnet ist, dass man eine Verbindung der allgemeinen Formel (I): 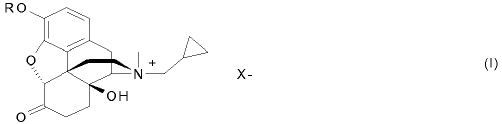 worin
X⁻ ein Anion, welches ausgewählt ist aus der Gruppe umfassend [-O-S(O)₂-CH₃]; [-O-S(O)₂-CF₃], [-O-S(O)₂-Phenyl], worin der Phenylring gegebenenfalls substituiert ist, und
R Wasserstoff oder eine Abgangsgruppe, bedeuten,
   (i) in einem geeigneten polaren Lösungsmittel, ausgewählt aus der Gruppe enthaltend Wasser, polare organische Lösungsmittel und aprotische dipolare Lösungsmittel, löst oder dispergiert,
   (ii) diese Lösung oder Dispersion mit einer Verbindung, welche Bromidanionen enthält, mischt, und das erhaltene Reaktionsgemisch so lange rührt, bis das N-Methylnaltrexonbromid gebildet und kristallisiert ist, wobei man für den Fall, dass R eine Abgangsgruppe bedeutet, diese während oder nach der Umsetzung entfernt.
X⁻ bedeutet vorzugsweise [-O-S(O)₂-CH₃]; [-O-S(O)₂-Phenyl], oder [-O-S(O)₂-Tolyl], vorzugsweise [-O-S(O)₂-CH₃].
R bedeutet vorzugsweise Wasserstoff oder eine Abgangsgruppe, vorzugsweise Wasserstoff. Für die Durchführung des erfindungsgemässen Austausches der Anionen ist die Anwesenheit der Schutzgruppe R, bzw. abspaltbare Abgangsgruppe R, nicht erforderlich. Die Verwendung einer Verbindung der allgemeinen Formel (I), worin R eine Abgangsgruppe bedeutet, ist aber dann sinnvoll, wenn diese Abgangsgruppe infolge der vorgängigen Methylierungsreaktion anwesend ist. Die Schutzgruppe kann dann auch nach der Austauschreaktion des Anions entfernt werden, wobei die Reaktionsbedingungen für die Entfernung der Abgangsgruppe an sich bekannt sind und je nach Schutzgruppe variieren. Man kann die erfindungsgemässe Reaktion zum Austausch der Anionen auch unter solchen Bedingungen durchführen, dass sich die Abgangsgruppe während der Austauschreaktion abspaltet. Dies ist dann möglich, wenn die Abgangsgruppe unter den Bedingungen des Ionenaustausches, z.B mit wässriger HBr, abgespalten werden kann.

Als Abgangsgruppe R verwendet man vorzugsweise (C₁-C₈)-Alkyloxycarbonyl oder Phenyloxycarbonyl; vorzugsweise Ethyloxycarbonyl, Isobutyloxycarbonyl, oder tert.-Butyloxycarbonyl (Boc), Cyclohexyloxycarbonyl, oder Trialkylsilyl; vorzugsweise (C₁-C₈)-Alkyloxycarbonyl oder Phenyloxycarbonyl; vorzugsweise Ethyloxycarbonyl oder tert.-Butyloxycarbonyl (Boc) oder Trialkylsilyl, wie beispielsweise Trimethylsilyl, ausgehend z.B. von (CH₃)₃SiCl oder Hexamethyldisilazan und andern an sich bekannten Organosilylverbindungen. Vorzugsweise bedeutet R: (C₁-C₈)-Alkyloxycarbonyl oder Phenyloxycarbonyl; vorzugsweise Ethyloxycarbonyl oder tert.-Butyloxycarbonyl (Boc).

Als Abgangsgruppe R können neben den genannten Schutzgruppen auch Estergruppierungen, wie z.B. Methylester-, Acetylester-, Benzylester-, oder gegebenenfalls substituierte Benzylestergruppen, verwendet werden.

Für die Einführung einer Alkyloxycarbonylgruppe, z.B. Ethyloxycarbonyl, verwendet man in an sich bekannter Weise Ethylchloroformiat. Die Verfahren zur Einführung von unterschiedlichen Schutzgruppen bzw. abspaltbaren Abgangsgruppen sind an sich aus der Literatur bekannt.

Die Verbindung der Formel (I) erhält man durch Methylierung der freien Naltrexonbase, was gleichzeitig zur Einführung des Anions X⁻ und zu einer Verbindung der allgemeinen Formel (I) führt. In diesem Sinne betrifft die vorliegende Erfindung ein Verfahren zur Herstellung der Verbindung der Formel (I), welches dadurch gekennzeichnet ist, dass man die freie Naltrexonbase mit einer den Substituenten X enthaltenden Verbindung umsetzt, wobei man gegebenenfalls vorgehend die phenolische Hydroxylgruppe mit einer Schutzgruppe versieht.

Beispiele solcher den Substituenten X⁻ enthaltenden Verbindungen sind: Methylchlorid (CH₃Cl); Methyliodid (CH₃I); Dimethylsulfat [CH₃-O-S(O)₂-O-CH₃]; Alkyl(C₁₋₄)sulfonsäuremethylester [CH₃-O-S(O)₂-(C₁₋₄)Alkyl]; vorzugsweise Methylsulfonsäuremethylester [CH₃-O-S(O)₂-CH₃]; Trifuormethylsulfonsäuremethylester [CH₃-O-S(O)₂-CF₃]; Phenylsulfonsäuremethylester [CH3-O-S(O)₂-Phenyl], worin der Phenylring gegebenenfalls substituiert ist, vorzugsweise in para-Stellung, vorzugsweise durch Methyl, vorzugsweise [CH₃-O-S(O)₂-CH₃]; [CH₃-O-S(O)₂-Phenyl], oder [CH₃-O-S(O)₂-Tolyl].

Vorzugsweise verwendet man für die Methylierung die genannten Sulfonatverbindungen, so dass die Substituenten X⁻ erhalten werden, wie diese hierin auch in den bevorzugten Ausführungsformen definiert sind.

Die Methylierung der freien Naltrexonbase, welche die Verbindung der allgemeinen Formel (I) ergibt, führt man vorzugsweise unter im wesentlichen wasserfreien Bedingungen durch. Bevorzugt sind diejenigen Verbindungen auch als Lösungsmittel, welche gleichzeitig die Methylgruppe für die Methylierung der freien Naltrexonbase sowie auch das erforderliche Anion (X⁻) enthalten, welches die Verbindung der allgemeinen Formel (I)ergibt. Diese bevorzugten Verbindungen entsprechen den bereits vorgehend genannten Verbindungen: wie Methylsulfonsäuremethylester, Trifuormethylsulfonsäuremethylester, Phenylsulfonsäuremethylester, worin der Phenylring gegebenenfalls substituiert ist, vorzugsweise in para-Stellung, vorzugsweise durch Methyl.

Bevorzugt für die Methylierung sind insbesondere die genannten Sulfonatverbindungen, welche die Substituenten X⁻ ergeben, wie diese hierin in den bevorzugten Ausführungsformen definiert sind.

Die erhaltene Verbindung der Formel (I) isoliert man vorzugsweise durch eine Fällungsreaktion, beispielsweise durch die Zugabe von Ethylacetat oder einer anderen Verbindung, welche mit dem Alkylierungsmittel mischbar ist. Beispiele für solche andere Verbindungen sind Ethylacetat, Methylacetat, tert.-Butylmethylether, Toluol, Tetrahydrofuran und 2-Methyltetrahydrofuran. Diese Liste lässt sich durch den Fachmann ohne weiteres mit Lösungsmitteln ähnlicher Polarität erweitern.

Die Verbindung der Formel (I) wird erfindungsgemäss in einem geeigneten polaren Lösungsmittel [Schritt (i)] gelöst oder dispergiert, wobei man dieses, die Verbindung der Formel (I) enthaltende polare Lösungsmittel, mit einer Verbindung, welche Bromidanionen enthält, mischt. Dabei wird das erhaltene Reaktionsgemisch so lange gerührt bis das N-Methylnaltrexonbromid gebildet und kristallisiert ist. So wird Methylnatrexonbromid durch Zugabe von Bromwasserstoff oder Zugabe von Bromiden, z.B. Natriumbromid, Kaliumbromid, Ammoniumbromid, gelöst in geeigneten polaren protischen Lösungsmittel, gewonnen. Geeignete polare Lösungsmittel zur Gewinnung und Fällung von Methylnatrexonbromid sind beispielsweise Wasser, sowie polare organische Lösungsmittel, wie aprotische dipolare Lösungsmittel wie Dimethylacetamid (DMA), Dimethylformamid (DMF), N-Methylpyrrolidon (NMP) oder Dimethylsulfoxid (DMSO), Alkohole, vorzugsweise aliphatische Alkohole. Bevorzugt sind die Systeme Wasser, Wasser/Methanol, Wasser/Ethanol, Dimethylformamid, Dimethylacetamid, N-Methylpyrrolidon, Acetonitril, Aceton/Dimethylformamid und Aceton/Dimethylacetamid, insbesondere aliphatische (C₁₋₄)-Alkohole oder ein Gemisch von solchen polaren organischen Lösungsmitteln mit Wasser.

Für die Fällungsreaktion verwendet man erfindungsgemäss stark saure Bedingungen, welche beispielsweise auch durch die wässrige Zersetzung des Alkylierungsmittels erhalten werden. Wasser liegt in der Regel, als Überschuss zum Zerstören des Alkylierungsmittels, in kleinen Mengen vor.

Bevorzugte Alkohole sind (C₁₋₄)-Alkohole, vorzugsweise Methanol, Ethanol, Propanol, Butanol, vorzugsweise Methanol. Dabei löst oder dispergiert man die Verbindung der Formel (I) in den genannten polaren Lösungsmitteln, vorzugsweise bei Raumtemperatur, vorzugsweise bei einer Temperatur im Bereich 20°C bis 80°C, vorzugsweise von 20°C bis 70°C, vorzugsweise von 20°C bis 65°C, vorzugsweise unter Rühren, wobei man die das Bromidanion enthaltende Verbindung zusetzt, und, vorzugsweise bei erhöhter Temperatur, so lange rührt bis sich das gewünschte N-Methylnaltrexonbromid in quantitativer Menge gebildet hat. Die Umsetzung beansprucht in der Regel eine Dauer von nur wenigen Minuten, beispielsweise etwa 5 Minuten, bis etwa 20 Stunden, vorzugsweise rührt man etwa 4-8 Stunden. Vorzugsweise kühlt man für die quantitative Isolierung des gebildeten Bromids das Reaktionsgemisch mindestens auf Raumtemperatur, vorzugsweise auf eine Temperatur im Bereich von Raumtemperatur (ca. 20°C) bis -30°C. Dabei wird die Konzentration der Verbindung der Formel (I) im Lösungsmittel derart gewählt, dass das gebildete N-Methylnaltrexonbromid in quantitativer Menge ausfällt, während die übrigen Komponenten gelöst bleiben. Dies ist eine Verfahrensoptimierung zur jeweiligen Festlegung der optimalen Konzentration der Verbindung der Formel (I) im jeweiligen Lösungsmittel und für den Fachmann problemlos.

Die Verbindung, welche mindestens ein Bromidanion enthält, kann man als solche der Lösung oder Dispersion der Verbindung der Formel (I) hinzufügen. Vorzugsweise löst man die Verbindung, welche mindestens ein Bromidanion enthält, in Wasser oder einem polaren organischen Lösungsmittel, wobei dieses polare Lösungsmittel ausgewählt ist aus der Gruppe enthaltend: Wasser, polare organische Lösungsmittel, aprotisch dipolare Lösungsmittel, vorzugsweise Dimethylacetamid (DMA), Dimethylformamid (DMF), N-Methylpyrrolidon (NMP) oder Dimethylsulfoxid (DMSO); Alkohole, vorzugsweise aliphatische Alkohole, vorzugsweise aliphatische (C₁₋₄)-Alkohole oder ein Gemisch von solchen polaren organischen Lösungsmitteln mit Wasser; vorzugsweise Wasser oder ein Gemisch von Wasser und Alkohol mit einem beliebigen Wassergehalt. Die Anwesenheit von Wasser ist für den Ionenaustausch nicht erforderlich und die Konzentration des allfällig anwesenden Wassers unkritisch.

Geeignete Verbindungen, welche Bromidanionen enthalten, sind sehr zahlreich und können anorganische oder organische Verbindungen sein. Natürlich kommen nur medizinisch zugelassene Verbindungen zur Anwendung. So können beispielsweise Schwermetallbromide, organische Bromide, Bromid abspaltende Phosphoniumsalze, und zahlreiche weitere Verbindungen verwendet werden. Bevorzugt sind Bromwasserstoffsäure (HBr); Alkalibromide, wie Natriumbromid oder Kaliumbromid; Erdalkalibromide, wie Calciumbromid; quaternäre, anorganische oder organische, Ammoniumbromide, wie Ammoniumbromid (NH₄Br) oder Dimethylammoniumbromid. Bevorzugt sind Natriumbromid und Dimethylammoniumbromid.

Die erfindungsgemässe Reaktion des Anionenaustausches führt man vorzugsweise bei einem Säurewert (pH-Wert) im Bereich von Null bis 9 (neun), vorzugsweise im Bereich von 2 (zwei) bis 5 (fünf) durch, wobei man diesen Säurewert vorzugsweise durch Zugabe von Bromwasserstoff (HBr) zum Reaktionsgemisch einstellt.

Erfindungsgemäss kann man die Methylierungsreaktion und den Austausch des durch die Methylierung erhaltenen Anions gegen das Bromidanion direkt nacheinander durchführen. In diesem Sinne betrifft die vorliegende Erfindung ein Verfahren zur Herstellung von N-Methylnaltrexonbromid, welches dadurch gekennzeichnet ist, dass man in einem ersten Schritt eine Verbindung der Formel (I) herstellt, indem man die freie Naltrexonbase mit einer den Substituenten X⁻ enthaltenden Verbindung umsetzt, wobei man gegebenenfalls vorgehend die phenolische Hydroxylgruppe der freien Natrexonbase mit einer Schutzgruppe versieht und anschliessend in einem zweiten Schritt die erhaltene Verbindung der allgemeinen Formel (I) in einem geeigneten polaren Lösungsmittel löst oder dispergiert, diese Lösung oder Dispersion mit einer Verbindung, welche Bromidanionen enthält, mischt, und das Reaktionsgemisch, bzw. die Lösung oder die Dispersion, so lange rührt, bis N-Methylnaltrexonbromid gebildet und kristallisiert ist, wobei man für den Fall, dass R eine Abgangsgruppe bedeutet, diese während oder nach der Umsetzung entfernt. Die Bedingungen für die Durchführung der einzelnen Schritte entsprechen den vorgehend genannten Bedingungen.

Gemäss einer besonderen Ausführungsform der vorliegenden Erfindung ist es auch möglich, sowohl die Methylierung als auch den Austausch des durch die Methylierung erhaltenen Anions gegen das Bromidanion, direkt nacheinander durchzuführen, ohne die Verbindung der Formel (I) aus dem Reaktionsgemisch zu isolieren (Eintopfverfahren). Wird für die Herstellung der Verbindung der Formel (I) als Methylierungsmittel ein Alkylsulfonsäuremethylester wie beispielsweise Methylsulfonsäuremethylester oder Trifuormethylsulfonsäuremethylester oder ein Phenylsulfonsäuremethylester verwendet, so ist es für das Eintopfverfahren notwendig, das überschüssige toxische Methylierungsmittel *in situ* zu zersetzen und eine gegebenenfalls anwesende, an die phenolische Gruppe gebundene, Schutzgruppe zu entfernen. Es wurde gefunden, dass beides überraschenderweise in Gegenwart von Wasser möglich ist, ohne dass das gebildete Sulfonat der Verbindung der Formel (I) Schaden nimmt. Dabei hat das Verfahren den Vorteil, dass die Zersetzung des überschüssigen toxischen Methylierungsmittels in neutralem oder saurem pH-Wert (pH ≤7), vorzugsweise bei saurem pH-Wert, durchgeführt werden kann, wobei die sauren Zersetzungsprodukte der Sulfonsäuremethylesterverbindung, einen vergleichsweise konstanten sauren pH-Wert während der Reaktionsführung bewirken.

Es ist jedoch auch möglich, die Zersetzung des überschüssigen toxischen Methylierungsmittels im Eintopfverfahren bei erhöhtem Säurewert (pH 8-12) durchzuführen, wobei man zur Erreichung des basischen pH-Werts dem Reaktionsgemisch eine Base, wie z.B. Natronlauge oder wässriges Ammoniak, zusetzt. Diese Ausführungsform hat jedoch den Nachteil, dass im Verfahren ein weiteres Reagenz zudosiert werden muss. Auch wird die Kristallisation des Methylnaltrexonbromids nach Zugabe der Bromidquelle vorzugsweise in saurem Medium durchgeführt.

Dabei entstehen aus dem überschüssigen toxischen Methylierungsmittel sowohl bei saurem als auch bei basischem pH-Wert, unbedenkliche Nebenprodukte. Im Reaktionsgemisch, enthaltend die Verbindung der Formel (I) und die untoxischen Nebenprodukte, kann dann durch Zugabe einer Bromidquelle, z.B. von wässrigem Wasserstoffbromid (HBr), die Verbindung der Formel (I) in Methylnaltrexonbromid umgewandelt werden. Dieses kristallisiert in reiner Form als Rohprodukt aus und kann gegebenenfalls umkristallisiert werden, wobei für die Kristallisation vorzugsweise ein saurer pH-Wert verwendet wird.

Das Eintopfverfahren ist besonders geeignet zur kostengünstigen Herstellung grosser Mengen Methylnaltrexonbromid, da die Isolierung des Methylnaltrexonbromids keine speziellen Sicherheitsvorkehrungen erfordert, nachdem, gemäss dieser Ausführungsform, der Dampfdruck des Methylierungsmittels in der Reaktionslösung, beispielsweise des verbleibenden Rests an toxischem Dimethylsulfat, weniger als 0.1 ppm (< 0.1 ppm), gemessen in der Dampfphase der Lösung bei 70°C, beträgt.

In diesem Sinne betrifft die vorliegende Erfindung ein Eintopfverfahren zur Herstellung von N-Methylnaltrexonbromid, welches dadurch gekennzeichnet ist, dass man eine Verbindung der allgemeinen Formel (I) herstellt, indem man die freie Naltrexonbase mit einem den Substituenten X⁻ enthaltenden Methylierungsmittel umsetzt, wie dies vorgehend beschrieben ist, wobei man gegebenenfalls vorgehend die phenolische Hydroxylgruppe der freien Natrexonbase mit einer Schutzgruppe versieht, und anschliessend das im Reaktionsgemisch vorhandene überschüssige Methylierungsreagenz in Gegenwart von Wasser, vorzugsweise bei neutralem oder saurem pH-Wert (pH ≤7), vorzugsweise bei saurem pH-Wert, und erhöhter Temperatur, zersetzt, so dass gleichzeitig eine gegebenenfalls vorhandene, an die phenolische Gruppe gebundene, Schutzgruppe entfernt wird; und anschliessend dem Reaktionsgemisch eine Verbindung, welche Bromidanionen enthält, zusetzt und so lange rührt, bis N-Methylnaltrexonbromid gebildet und kristallisiert ist.

Die vorliegende Erfindung betrifft auch das vorgehend definierte Eintopfverfahren zur Herstellung von N-Methylnaltrexonbromid, welches dadurch gekennzeichnet ist, dass man die Zersetzung des im Reaktionsgemisch vorhandenen überschüssigen Methylierungsmittels bei erhöhtem Säurewert (pH 8-12) durchführt, wobei man zur Erreichung des basischen pH-Werts dem Reaktionsgemisch eine Base, wie z.B. Natronlauge oder wässriges Ammoniak, zusetzt. Die Kristallisation des N-Methylnaltrexonbromids, führt man anschliessend vorzugsweise in saurem Medium durch.

Dabei setzt man für die Zersetzung des im Reaktionsgemisch vorhandenen überschüssigen Methylierungsmittels vorzugsweise mindestens ein Äquivalent Wasser bezogen auf den Überschuss an Alkylierungsmittel ein. Bevorzugt ist ein Überschuss an Wasser, beispielsweise 1.1 bis 5.0 Äquivalente Wasser oder einem anderen Reagenz, welches das Alkylierungsmittel zerstört, bezogen auf den Überschuss an noch vorhandenem Alkylierungsmittel, und erhitzt vorzugsweise das Reaktionsgemisch auf eine Temperatur im Bereich von 50°C bis 90°C, vorzugsweise auf 70°C bis 80°C während 4-8 Stunden. Unter diesen Bedingungen werden das überschüssige Alkylierungsmittel zerstört und allfällig vorhandene Abgangsgruppen entfernt.

Für das Eintopfverfahren geht man erfindungsgemäss vorzugsweise so vor, dass man beispielsweise Naltrexonalkylethylcarbonat methyliert, vorzugsweise über einen Zeitraum von etwa 10 bis 20 Stunden, bei einer Temperatur von vorzugsweise etwa 50°C bis 60°C. Anschliessend wird durch Zugabe von Wasser zum Reaktionsgemisch und Erwärmung desselben auf etwa 70°C bis 80°C, während etwa 4 bis 8 Stunden, das überschüssige Methylierungsmittel zersetzt, bis der Messwert im Dampfdruck der Lösung kleiner ist als 0.1 ppm. Dann wird dem Reaktionsgemisch Methanol und wässrige HBr-Lösung zugegeben, wobei sich Methylnaltrexonbromid bildet, welches auskristallisiert.

Vorzugsweise wird jeweils das derart erhaltene N-Methylnaltrexonbromid Rohprodukt aus einem geeigneten Lösungsmittel, vorzugsweise aus Wasser oder wässerigem Alkohol, vorzugsweise aus wässerigem Alkohol, vorzugsweise aus Ethanol oder Methanol, vorzugsweise aus Methanol, enthaltend 1% bis 99% Wasser, berechnet auf das Gesamtgewicht von Wasser und Methanol, umkristallisiert. Dabei wird bei einer Umkristallisation aus Wasser/Methanol oder Wasser ein Wert des Bromidgehalts erreicht, der zu mindestens 99.99% (>99.99%) mit der theoretisch berechneten Menge an Bromidionen im Methylnaltrexonbromid übereinstimmt. Vorzugsweise kristallisiert man aus einer gesättigten Lösung, was dem Fachmann bekannt ist.

Die vorliegende Erfindung betrifft auch die Verwendung des erfindungsgemäss hergestellten N-Methylnaltrexonbromids als Heilmittel und die Verwendung der erfindungsgemäss hergestellten N-Methylnaltrexonbromids zur Herstellung eines pharmazeutisch verabreichbaren Heilmittels, insbesondere zur Behandlung von postoperativem Ileus oder zur Behandlung von Opioid-induzierter Konstipation. Die folgenden Beispiele erläutern die Erfindung ohne diese zu beschränken.

### Beispiel 1 (Herstellung Naltrexon/Einführung Schutzgruppe)

a) 100g Noroxymorphon werden in 165g N-Methylpyrrolidinon und 37g Natriumcarbonat suspendiert und mit 56g Brommethylcyclopropan versetzt. Die Suspension wird auf 70°C für 3 Stunden erwärmt. Danach wird abgekühlt und das Produkt mittels 803g Wasser kristallisiert. Dabei wird mit Naltrexon angeimpft. Am Ende der Wasserzugabe wird der pH-Wert mittels Zugabe von Natronlauge auf 9.5 eingestellt. Das Produkt wird abgesaugt, mit Wasser gewaschen und im Vakuum getrocknet. Ausbeute: 113g an Naltrexon roh, Reinheit > 95%.
b) 50g Naltrexon roh und 1.7g Aktivkohle werden in 400g Ethylacetat für 1 Stunde zum Rückfluss erhitzt. Danach wird die Aktivkohle abfiltriert. Die Hälfte der Menge an Ethylacetat wird abdestilliert. Es werden 21g Triethylamin und 18g Chlorameisensäureethylester zugeben und die Mischung 1 Stunde bei 45°C erwärmt, anschliessend 100g Wasser hinzugefügt und die Phasen getrennt. Die organische Phase wird nochmals mit 100g Wasser gewaschen und dann bei Normaldruck bis auf ein Minimum eingeengt. Zur Vervollständigung der Kristallisation werden 218g Isopropanol hinzugegeben. Die Suspension wird auf 0°C abgekühlt. Das Produkt wird abgesaugt, mit Isopropanol gewaschen und im Vakuum getrocknet. Ausbeute: 53g Naltrexoncarbonat, Reinheit > 99.3%

### Beispiel 2 (Methylierung mit Trifluormethansulfonat)

30g Naltrexonethylcarbonat werden in 50g Trifluormethan-sulfonsäuremethylester gelöst und 8 Stunden bei 40°C gerührt. Dann werden 30g Ethylacetat zugegeben. Das ausgefallene Produkt wird abgesaugt mit Ethylacetat nachgewaschen und im Vakuum getrocknet. Es werden 37.8g Methylnaltrexonethylcarbonattrifluormethansulfonat mit einer Reinheit von >96% isoliert.

### Beispiel 3 (Methylierung mit p-Toluolsulfonsäuremethylester)

30g Naltrexonethylcarbonat werden in 50g Dimethylacetamid gelöst und mit 30g p-Toluolsulfonsäuremethylester 48 Stunden bei 60°C gerührt. Dann werden 40g Ethylacetat zugegeben. Das ausgefallene Produkt wird abgesaugt mit Ethylacetat nachgewaschen und im Vakuum getrocknet. Es werden 33.2g Methylnaltrexonethylcarbonat-p-toluolsulfonat mit einer Reinheit von >96% isoliert.

## Patentansprüche

1. Verfahren zur Herstellung von N-Methylnaltrexonbromid, **dadurch gekennzeichnet, dass** man eine Verbindung der allgemeinen Formel (I): 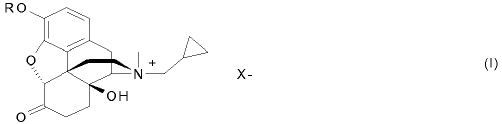
X⁻ ein Anion, welches ausgewählt ist aus der Gruppe umfassend [-O-S(O)₂-CH₃], [-O-S(O)₂-CF₃], [-O-S(O)₂-Phenyl], worin der Phenylring gegebenenfalls substituiert ist, und
R Wasserstoff oder eine Abgangsgruppe, bedeuten,
(i) in einem geeigneten polaren Lösungsmittel, ausgewählt ist aus der Gruppe enthaltend Wasser, polare organische Lösungsmittel und aprotische dipolare Lösungsmittel, löst oder dispergiert,
(ii) diese Lösung oder Dispersion mit einer Verbindung, welche Bromidanionen enthält, mischt, und das erhaltene Reaktionsgemisch so lange rührt, bis das N-Methylnaltrexonbromid gebildet und kristallisiert ist, wobei man für den Fall, dass R eine Abgangsgruppe bedeutet, diese während oder nach der Umsetzung entfernt.

2. Verfahren nach Anspruch 1, **dadurch gekennzeichnet, dass** X⁻:
[-O-S(O)₂-CH₃]; [-O-S(O)₂-Phenyl], oder [-O-S(O)₂-Tolyl] bedeutet.

3. Verfahren nach Anspruch 1, **dadurch gekennzeichnet, dass** X⁻:
[-O-S(O)₂-CH₃] bedeutet.

4. Verfahren nach einem der Ansprüche 1-4, **dadurch gekennzeichnet, dass** R Wasserstoff bedeutet.

5. Verfahren nach einem der Ansprüche 1-4, **dadurch gekennzeichnet, dass** R eine Abgangsgruppe bedeutet, welche ausgewählt ist aus (C₁-C₈)-Alkyloxycarbonyl oder Phenyloxycarbonyl oder Trialkylsilyl.

6. Verfahren nach Anspruch 5, **dadurch gekennzeichnet, dass** R eine Abgangsgruppe bedeutet, welche ausgewählt ist aus Ethyloxycarbonyl, Isobutyloxycarbonyl, tert.-Butyloxycarbonyl (Boc), oder Cyclohexyloxycarbonyl; vorzugsweise Ethyloxycarbonyl oder tert.-Butyloxycarbonyl (Boc).

7. Verfahren nach einem der Ansprüche 1-4, **dadurch gekennzeichnet, dass** R eine Abgangsgruppe bedeutet, welche ausgewählt ist aus der Gruppe umfassend Estergruppierungen, vorzugsweise Methylester-, Acetylester-, Benzylester-, oder gegebenenfalls substituierte Benzylestergruppen.

8. Verfahren nach einem der Ansprüche 1-7, **dadurch gekennzeichnet, dass** das polare Lösungsmittel ausgewählt ist aus der Gruppe enthaltend: Dimethylacetamid (DMA), Dimethylformamid (DMF), N-Methylpyrrolidon (NMP) oder Dimethylsulfoxid (DMSO); Alkohole, vorzugsweise aliphatische Alkohole, vorzugsweise aliphatische (C₁₋₄)-Alkohole.

9. Verfahren nach Anspruch 8, **dadurch gekennzeichnet, dass** das Lösungsmittel ausgewählt ist aus der Gruppe enthaltend: Wasser, Wasser/Methanol, Wasser/Ethanol, Dimethylformamid, Dimethylacetamid, N-Methylpyrrolidon, Acetonitril, Aceton/- Dimethylformamid und Aceton/Dimethylacetamid, vorzugsweise aliphatische (C₁₋₄)-Alkohole oder ein Gemisch von solchen polaren organischen Lösungsmitteln mit Wasser.

10. Verfahren nach einem der Ansprüche 1-9, **dadurch gekennzeichnet, dass** man die Verbindung der Formel (I) mit einer Verbindung, welche Bromidanionen enthält, umsetzt, und diese Bromidanionen enthaltende Verbindung vorzugsweise in Wasser oder einem polaren organischen Lösungsmittel gelöst ist, und das polare Lösungsmittel ausgewählt ist aus der Gruppe enthaltend:
polare organische Lösungsmittel, aprotische dipolare Lösungsmittel, vorzugsweise Dimethylacetamid (DMA), Dimethylformamid (DMF), N-Methylpyrrolidon (NMP) oder Dimethylsulfoxid (DMSO);
Alkohole, vorzugsweise aliphatische Alkohole, vorzugsweise aliphatische (C₁₋₄)-Alkohole oder ein Gemisch von solchen polaren organischen Lösungsmitteln mit Wasser; vorzugsweise Wasser oder ein Gemisch von Wasser und Alkohol mit einem beliebigen Wassergehalt.

11. Verfahren nach einem der Ansprüche 1-10, **dadurch gekennzeichnet, dass** die Verbindung, welche Bromidanionen enthält, ausgewählt ist aus anorganischen oder organischen medizinisch zugelassenen Verbindungen, vorzugsweise Bromwasserstoffsäure (HBr); Alkalibromide, vorzugsweise Natriumbromid oder Kaliumbromid; Erdalkalibromide, vorzugsweise Calciumbromid; quaternäre, anorganische oder organische, Ammoniumbromide, vorzugsweise Ammoniumbromid (NH₄Br) oder Dimethylammoniumbromid; vorzugsweise Natriumbromid und Dimethylammoniumbromid.

12. Verfahren nach einem der Ansprüche 1-11, **dadurch gekennzeichnet, dass** man die Reaktion des Anionenaustausches bei einem Säurewert (pH-Wert) im Bereich von Null bis 9 (neun), vorzugsweise im Bereich von 2 (zwei) bis 5 (fünf) durchführt, wobei man diesen Säurewert vorzugsweise durch Zugabe von Bromwasserstoff (HBr) zum Reaktionsgemisch einstellt.

13. Verfahren zur Herstellung von N-Methylnaltrexonbromid, **dadurch gekennzeichnet, dass** man in einem ersten Schritt eine Verbindung der Formel (I) herstellt, indem man die freie Naltrexonbase mit einer den Substituenten X enthaltenden Verbindung umsetzt, wobei man gegebenenfalls vorgehend die phenolische Hydroxylgruppe der freien Natrexonbase mit einer Abgangsgruppe gemäss der Definition in den Ansprüchen 5, 6 oder 7 versieht und anschliessend in einem zweiten Schritt die erhaltene Verbindung der allgemeinen Formel (I) in einem geeigneten polaren Lösungsmittel, gemäss der Definition in den Ansprüchen 1, 8 oder 9, löst oder dispergiert, diese Lösung oder Dispersion mit einer Verbindung, welche Bromidanionen gemäss der Definition in den Ansprüchen 10 oder 11 enthält, mischt, und das Reaktionsgemisch, bzw. die Lösung oder die Dispersion, so lange rührt, bis N-Methylnaltrexonbromid gebildet und kristallisiert ist, wobei man für den Fall, dass R eine Abgangsgruppe bedeutet, diese während oder nach der Umsetzung entfernt.

14. Verfahren nach Anspruch 13, **dadurch gekennzeichnet, dass** die den Substituenten X enthaltenden Verbindung ausgewählt ist aus der Gruppe enthaltend: [CH₃-O-S(O)₂-CH₃]; [CH₃-O-S(O)₂-CF₃], [CH₃-O-S(O)₂-Phenyl], worin der Phenylring gegebenenfalls substituiert ist, vorzugsweise [CH₃-O-S(O)₂-CH₃]; [CH₃-O-S(O)₂-Phenyl], oder [CH₃-O-S(O)₂-Tolyl].

15. Verfahren nach Anspruch 14 oder 15, **dadurch gekennzeichnet, dass** man die erhaltene Verbindung der Formel (I), mittels einer Fällungsreaktion isoliert, vorzugsweise durch die Zugabe einer Verbindung, welche mit dem Alkylierungsmittel mischbar ist, vorzugsweise durch Zugabe von Ethylacetat, Methylacetat, tert.-Butylmethylether, Toluol, Tetrahydrofuran und 2-Methyltetrahydrofuran.

16. Verfahren zur Herstellung von N-Methylnaltrexonbromid als Eintopfverfahren, **dadurch gekennzeichnet, dass** man eine Verbindung der allgemeinen Formel (I) herstellt, indem man die freie Naltrexonbase mit einem den Substituenten X enthaltenden Methylierungsmittel gemäss der Definition in Anspruch 14 umsetzt, wobei man gegebenenfalls vorgehend die phenolische Hydroxylgruppe der freien Natrexonbase mit einer Schutzgruppe versieht, und anschliessend, das im Reaktionsgemisch vorhandene überschüssige Methylierungsmittel in Gegenwart von Wasser, vorzugsweise bei neutralem oder saurem pH-Wert (pH ≤7), vorzugsweise bei saurem pH-Wert, und erhöhter Temperatur, zersetzt, so dass gleichzeitig eine gegebenenfalls vorhandene, an die phenolische Gruppe gebundene, Schutzgruppe entfernt wird; und anschliessend dem Reaktionsgemisch eine Verbindung, welche Bromidanionen enthält, zusetzt und so lange rührt, bis N-Methylnaltrexonbromid gebildet und kristallisiert ist.

17. Verfahren nach Anspruch 16, **dadurch gekennzeichnet, dass** man die Zersetzung des im Reaktionsgemisch vorhandenen überschüssigen Methylierungsmittels bei erhöhtem Säurewert (pH 8-12) durchführt, wobei man zur Erreichung des basischen pH-Werts dem Reaktionsgemisch eine Base, vorzugsweise Natronlauge oder wässriges Ammoniak, zusetzt.

18. Verfahren nach einem der Ansprüche 1-17, **dadurch gekennzeichnet, dass** man das erhaltene N-Methylnaltrexonbromid Rohprodukt aus einem geeigneten Lösungsmittel, vorzugsweise aus Wasser oder wässerigem Alkohol, vorzugsweise aus wässerigem Alkohol, vorzugsweise aus Ethanol oder Methanol, vorzugsweise aus Methanol, enthaltend 1% bis 99% Wasser, berechnet auf das Gesamtgewicht von Wasser und Methanol, umkristallisiert.

## Claims

1. Method for production of N-methylnaltrexone bromide, **characterized in that** a compound of the general formula (I): 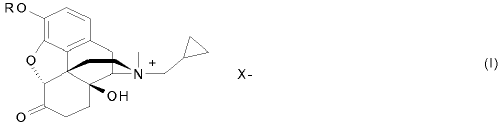 wherein
X⁻ represents an anion which is selected from the group comprising [-O-S(O)₂-CH₃], [-O-S(O)₂-CF₃], [-O-S(O)₂-phenyl], wherein the phenyl ring is optionally substituted, and R represents hydrogen or a leaving group,
(i) is dissolved or dispersed in a suitable polar solvent selected from the group including water, polar organic solvents, aprotic dipolar solvents,
(ii) this solution or dispersion is mixed with a compound containing bromide anions, and the reaction mixture obtained is stirred until N-methylnaltrexone bromide is formed and crystallized, wherein, in the case where R represents a leaving group, this is removed during or after the reaction.

2. Method according to claim 1, **characterized in that** X⁻ represents [-O-S(O)₂-CH₃]; [-O-S(O)₂-phenyl], or [-O-S(O)₂-tolyl].

3. Method according to claim 1, **characterized in that** X⁻ represents [-O-S(O)₂-CH₃].

4. Method according to one of claims 1 to 4, **characterized in that** R represents hydrogen.

5. Method according to one of claims 1 to 4, **characterized in that** R represents a leaving group selected from the group consisting of (C₁-C₈)-alkyloxycarbonyl or phenyloxycarbonyl or trialkylsilyl.

6. Method according to claim 5, **characterized in that** R represents a leaving group selected from ethyloxycarbonyl, isobutyloxycarbonyl, tert-butyloxycarbonyl (Boc), or cyclohexyloxycarbonyl; preferably ethyloxycarbonyl or tert-butyloxycarbonyl (Boc).

7. Method according to one of claims 1 to 4, **characterized in that** R represents a leaving group selected from the group comprising ester groupings, preferably methyl ester, acetyl ester, benzyl ester or optionally substituted benzyl ester groups.

8. Method according to one of claims 1 to 7, **characterized in that** the polar solvent is selected from the group including: dimethylacetamide (DMA), dimethyl formamide (DMF), N-methylpyrrolidone (NMP) or dimethyl sulfoxide (DMSO); alcohols, preferably aliphatic alcohols, preferably aliphatic (C₁₋₄) alcohols.

9. Method according to claim 8, **characterized in that** the solvent is selected from the group including: water, water/methanol, water/ethanol, dimethyl formamide, dimethylacetamide, N-methylpyrrolidone, acetonitrile, acetone/dimethyl formamide and acetone/dimethylacetamide, preferably aliphatic (C₁₋₄) alcohols or a mixture of such polar organic solvents with water.

10. Method according to one of claims 1 to 9, **characterized in that** the compound of formula (I) is reacted with a compound containing bromide anions, and this compound containing bromide anions is preferably dissolved in water or a polar organic solvent, and the polar solvent is selected from the group containing: polar organic solvents, aprotic dipolar solvents, preferably dimethylacetamide (DMA), dimethyl formamide (DMF), N-methylpyrrolidone (NMP) or dimethylsulfoxide (DMSO); alcohols, preferably aliphatic alcohols, preferably aliphatic (C₁₋₄) alcohols or a mixture of such polar organic solvents with water; preferably water or a mixture of water and alcohol with any water content.

11. Method according to one of claims 1 to 10, **characterized in that** the compound containing bromide anions is selected from the group consisting of inorganic and organic medically approved compounds, preferably hydrobromic acid (HBr); alkali bromides, preferably sodium bromide or potassium bromide; alkaline earth bromides, preferably calcium bromide; quaternary, inorganic or organic ammonium bromides, preferably ammonium bromide (NH₄Br) or dimethylammonium bromide; preferably sodium bromide and dimethylammonium bromide.

12. Method according to one of claims 1 to 11, **characterized in that** the anion exchange reaction is carried out at an acid value (pH value) in the range from zero to 9 (nine), preferably in the range from 2 (two) to 5 (five), this acid value being set by addition of hydrogen bromide (HBr) to the reaction mixture.

13. Method for producing N-methylnaltrexone bromide, **characterized in that** in a first step a compound of formula (I) according to claim 1 is produced by reacting the free naltrexone base with a compound containing the substituent X, wherein the phenolic hydroxyl group of the free naltrexone base is optionally provided beforehand with a leaving group according to the definition in claims 5, 6 or 7, and then in a second step the resulting compound of the general formula (I) is dissolved or dispersed in a suitable polar solvent, according to the definition in claims 1, 8 or 9, this solution or dispersion is mixed with a compound containing bromide anions according to the definition in claims 10 or 11, and the reaction mixture or the solution or dispersion is stirred until N-methylnaltrexone bromide is formed and crystallized, wherein, in the case where R represents a leaving group, this group is removed during or after the reaction.

14. Method according to claim 13, **characterized in that** the compound containing the substituent X is selected from the group including: [CH₃⁻O⁻S(O)₂⁻CH₃]; [CH₃⁻O⁻S(O)₂⁻CF₃], [CH₃⁻O⁻S(O)₂⁻phenyl], wherein the phenyl is potentially substituted, preferably [CH₃⁻O-S(O)₂⁻CH₃]; [CH₃-O-S(O)₂-phenyl], or [CH₃-O-S(O)₂-tolyl].

15. Method according to claim 14 or 15, **characterized in that** the resulting compound of formula (I) is isolated by means of a precipitation reaction, preferably by the addition of a compound which is miscible with the alkylating agent, preferably ethyl acetate, methyl acetate, tert.-butyl methyl ether, toluene, tetrahydrofuran and 2-methyltetrahydrofuran.

16. Method for producing N-methylnaltrexone bromide as a one-pot process, **characterized in that** a compound of the general formula (I) is produced, by reacting the free naltrexone base with a methylating agent containing the substituent X according to the definition in claim 14, wherein the phenolic hydroxyl group of the free naltrexone base is optionally provided beforehand with a protecting group, and then the excess methylating agent present in the reaction mixture is decomposed in the presence of water, preferably at a neutral or acidic pH value (pH≤7), preferably at an acidic pH value, and at an elevated temperature, so that simultaneously a protecting group which may be present bonded to the phenolic group is removed; and then a compound containing bromide anions is added to the reaction mixture and is stirred until N-methylnaltrexone bromide is formed and crystallized.

17. Method according to claim 16, **characterized in that** the decomposition of the excess methylating agent present in the reaction mixture is carried out at an elevated acid value (pH 8 to 12), wherein a base, preferably sodium hydroxide solution or aqueous ammonia, is added to the reaction mixture in order to achieve the basic pH value.

18. Method according to one of claims 1 to 17, **characterized in that** the resulting N-methylnaltrexone bromide crude product is recrystallized from a suitable solvent, preferably from water or aqueous alcohol, preferably from ethanol or methanol, preferably from methanol, containing from 1% to 99% water, based on the total weight of water and methanol.

## Revendications

1. Procédé de préparation du bromure de N-méthylnaltrexone, **caractérisé en ce qu'**un composé de la formule générale (I) : 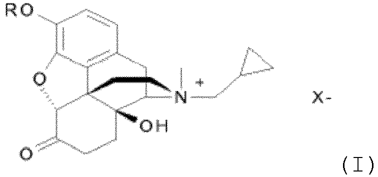 X⁻ représentant un anion qui est choisi dans le groupe constitué par [-O-S(O)₂-CH₃], [-O-S(O)₂-CF₃], [-O-S(O)₂-phényle], où le noyau phényle est éventuellement substitué, et R représentant un atome d'hydrogène ou un groupe partant, est dissous ou dispersé (i) dans un solvant polaire approprié choisi dans le groupe constitué par l'eau, les solvants organiques polaires et les solvants dipolaires aprotiques, (ii) cette solution ou dispersion est mélangée à un composé qui contient des anions bromure, et le mélange réactionnel résultant est agité jusqu'à ce que le bromure de N-méthylnaltrexone soit formé et cristallisé ; dans le cas où R est un groupe partant, celui-ci est éliminé pendant ou après la réaction.

2. Procédé selon la revendication 1, **caractérisé en ce que** X⁻ représente [-O-S(O)₂-CH₃] ; [-O-S(O)₂-phényle], ou [-0-S(O)₂-tolyl].

3. Procédé selon la revendication 1, **caractérisé en ce que** X⁻ représente [-O-S(O)₂-CH₃].

4. Procédé selon l'une des revendications 1 à 4, **caractérisé en ce que** R représente l'hydrogène.

5. Procédé selon l'une des revendications 1 à 4, **caractérisé en ce que** R est un groupe partant qui est choisi parmi alkyl (C₁ à C₈)-oxycarbonyle ou phényloxycarbonyle ou trialkylsilyle.

6. Procédé selon la revendication 5, **caractérisé en ce que** R représente un groupe partant qui est choisi parmi éthyloxycarbonyle, isobutyloxycarbonyle, tert.-butyloxycarbonyle (Boc), ou cyclohexyloxycarbonyle ; de préférence, éthyloxycarbonyle ou tert.-butyloxycarbonyle (Boc).

7. Procédé selon l'une des revendications 1 à 4, **caractérisé en ce que** R représente un groupe partant qui est choisi dans le groupe constitué par des groupes ester, de préférence des groupes ester méthylique, ester acétylique, ester benzylique, ou éventuellement ester benzylique substitué.

8. Procédé selon l'une des revendications 1 à 7, **caractérisé en ce que** le solvant polaire est choisi dans le groupe comprenant : le diméthylacétamide (DMA), le diméthylformamide (DMF), le N-méthylpyrrolidone (NMP) ou le diméthylsulfoxyde (DMSO) ; des alcools, de préférence les alcools aliphatiques, de préférence les alcools aliphatiques en C₁ à C₄.

9. Procédé selon la revendication 8, **caractérisé en ce que** le solvant est choisi dans le groupe comprenant : l'eau, l'eau/méthanol, l'eau/éthanol, le diméthylformamide, le diméthylacétamide, la N-méthylpyrrolidone, l'acétonitrile, l'acétone/diméthylformamide et l'acétone/diméthylacétamide, de préférence des alcools aliphatiques en C₁ à C₄ ou un mélange de tels solvants organiques polaires avec de l'eau.

10. Procédé selon l'une des revendications 1 à 9, **caractérisé en ce que** le composé de la formule (I) est mis à réagir avec un composé qui contient des anions bromure et ce composé contenant des anions bromure est de préférence dissous dans l'eau ou dans un solvant organique polaire, et le solvant polaire est choisi dans le groupe comprenant : un solvant organique polaire, un solvant aprotique dipolaire, de préférence le diméthylacétamide (DMA), le diméthylformamide (DMF), la N-méthylpyrrolidone (NMP) ou le diméthylsulfoxyde (DMSO) ; les alcools, de préférence les alcools aliphatiques, de préférence les alcools aliphatiques en C₁ à C₄ ou un mélange de tels solvants organiques polaires avec de l'eau ; de préférence de l'eau ou un mélange d'alcool et d'eau avec une teneur en eau quelconque.

11. Procédé selon l'une des revendications 1 à 10, **caractérisé en ce que** le composé qui contient des anions bromure est choisi parmi les composés médicalement approuvés minéraux ou organiques, de préférence l'acide bromhydrique (HBr) ; les bromures d'alcalins, de préférence le sodium ou le potassium ; les bromures d'alcalino-terreux, de préférence le calcium ; les bromures d'ammonium quaternaire minéraux ou organiques, de préférence le bromure d'ammonium (NH₄Br) ou le bromure de diméthylammonium, de préférence le bromure de sodium et le bromure de diméthylammonium.

12. Procédé selon l'une des revendications 1 à 11, **caractérisé en ce que** la réaction de l'échange d'anions est effectuée à un indice d'acidité (pH) dans la gamme allant de zéro à 9 (neuf), de préférence dans la gamme allant de 2 (deux) à 5 (cinq), ces indices d'acidité étant ajustées de préférence par addition de bromure d'hydrogène (HBr) au mélange réactionnel.

13. Procédé de préparation de bromure de N-méthylnaltrexone, **caractérisé en ce que**, dans une première étape, un composé de la formule (I) est préparé par réaction de la base naltrexone libre avec le composé contenant le substituant X, le groupe hydroxyle phénolique de la base libre natrexone étant éventuellement pourvu auparavant d'un groupe partant selon la définition des revendications 5, 6 ou 7 puis, dans une seconde étape, le composé obtenu de la formule générale (I) est dissous ou dispersé dans un solvant polaire approprié selon la définition des revendications 1, 8 ou 9, cette solution ou dispersion est mélangée à un composé qui contient des anions bromure selon la définition des revendications 10 ou 11, et le mélange réactionnel ou la solution ou la dispersion est agité jusqu'à ce que le bromure de N-méthylnaltrexone soit formé et cristallisé ; dans le cas où R est un groupe partant, celui-ci est éliminé pendant ou après la réaction.

14. Procédé selon la revendication 13, **caractérisé en ce que** le composé contenant le substituants X est choisi dans le groupe constitué par : [CH₃-O-S(O)₂-CH₃] ; [CH₃-O-S(O)₂-CF₃], (CH₃-O-S(O)₂-phényle], où le cycle phényle est éventuellement substitué, de préférence [CH₃-O-S(O)₂-CH₃] ; [CH₃-O-S(O)₂-phényl] ou [CH₃-O-S(O)₂-tolyl].

15. Procédé selon la revendication 14 ou 15, **caractérisé en ce que** le composé obtenu de la formule (I) est isolé par une réaction de précipitation, de préférence par addition d'un composé qui est miscible avec l'agent d'alkylation, de préférence par addition d'acétate d'éthyle, d'acétate de méthyle, de tert.-butylméthyléther, de toluène, de tétrahydrofuranne et de 2-méthyltétrahydrofuranne.

16. Procédé de préparation de bromure de N-méthylnaltrexone comme un procédé monotope, **caractérisé en ce qu'**un composé de la formule générale (I) est préparé par réaction de la base libre naltrexone avec un agent de méthylation, contenant le substituant X, selon la définition de la revendication 14, le groupe hydroxyle phénolique de la base libre natrexone étant éventuellement pourvu auparavant d'un groupe de protection, puis l'agent de méthylation en excès dans le mélange réactionnel est décomposé en présence d'eau, de préférence à un pH neutre ou acide (pH ≤ 7), de préférence un pH acide, et à une température élevée de façon à éliminer dans le même temps un groupe de protection éventuellement présent qui est lié au groupe phénolique ; puis le mélange réactionnel est additionné d'un composé contenant des anions bromure et agité jusqu'à former et cristalliser le bromure de N-méthylnaltrexone.

17. Procédé selon la revendication 16, **caractérisé en ce que** la décomposition de l'agent de méthylation en excès présent dans le mélange réactionnel est effectuée avec un indice d'acidité majorée (pH 8 à 12), une base, de préférence de l'hydroxyde de sodium ou l'ammoniaque, étant ajoutée au mélange réactionnel pour obtenir le pH basique.

18. Procédé selon l'une des revendications 1 à 17, **caractérisé en ce que** le produit brut bromure de N-méthylnaltrexone obtenu à partir d'un solvant approprié, de préférence de l'eau ou un alcool aqueux, de préférence l'alcool aqueux, de préférence de l'éthanol ou du méthanol, de préférence du méthanol, contenant 1% à 99% d'eau, calculé sur le poids total d'eau et de méthanol, est recristallisé.
